# EUROPÄISCHE PATENTANMELDUNG

(11) **EP 2 314 666 A1**
(43) Veröffentlichungstag der Anmeldung: **27.04.2011**
(21) Anmeldenummer: 10013957.5
(22) Anmeldetag: 21.10.2010
(51) Int. Cl.: C12M 1/107

(54) **Verfahren und Vorrichtung zur Erhöhung der Besiedlungsdichte von methanbildenden Bakterienstämmen in Biogasfermentern**

(30) Priorität: 21.10.2009 DE 102009045895
(71) Anmelder: PRV -- Planungsbüro Rossow - Gesellschaft Für Versorgungstechnik MBH, 17033 Neubrandenburg (DE)
(72) Erfinder: Rossow, Norbert, 17217 Penzlin (DE)
(74) Vertreter: Wehlan, Helmut

(57) **Zusammenfassung**

Die Erfindung betrifft einen Biogasfermenter mit Einrichtungen und Substraten zur Erzeugung von Biogas, wobei sich auf oder unterhalb des oberen Substrat-Flüssigkeitsstandes eine die gesamte Oberfläche umfassende Schwimmschicht - vorzugsweise aus grobfaserigem Material - befindet, in der methanbildende Bakterien immobilisiert sind.

## Beschreibung

Die Erfindung bezieht sich auf ein Verfahren und die Vorrichtung einer gezielten Einbringung und Kultivierung einer Schwimmschicht zur Erhöhung und Steuerung der Besiedlungsdichte von methanbildenden Bakterienstämmen in Biogasfermentern. Indem in die jeweils energetisch umzusetzenden Gärsubstrate zusätzlich grobfaserige Materialien eingeleitet, hieraus separate Schichten für eine Bakterienansiedlung und -konzentration geschaffen und durch die spezifische Anlagenkonfiguration - abhängig von der Fermenterorganik - optimiert werden, beschleunigt sich die Substratumsetzung, und die Schwimmschicht ersetzt physikalische Einrichtungen, welche die Steigerung der Raumbelastung ermöglichen sowie den Austrag von methanbildenden Substraten aus dem quasikontinuierlichen Fermenter weitestgehend mindern.

Bekanntlich ist die Konfiguration von Biogasanlagen, darunter der Fermenter, in nicht geringem Maße abhängig von den zum Einsatz gelangenden Substraten. Aus natürlichen, energetischen und betriebswirtschaftlichen Erwägungen geht der Trend zu einer entsprechenden Spezialisierung und der Entwicklung sowie dem Einsatz von spezifischen Hochleistungsreaktoren. Diese zeichnen sich u.a. durch kompakte Bauweise, geringes Volumen, eine höhere Raumbelastung, damit verbunden raschere Substratausbeute und geringere Verweilzeiten aus. Eine schnellere Zuführung neuen bzw. Abführung abgebauten Substrats aber hat zur Folge, dass methanbildende Bakterien partiell mit ausgespült werden, ohne genügend Zeit für die erforderliche ausreichende Reproduktion zu haben. Dies gilt es genauso zu vermeiden wie den vorzeitigen Austrag von methanisierungsfähigen Inhaltsstoffen. Besondere Bedeutung hat dies bei Inputstoffen mit geringem TS-Gehalt (TS = Trockensubstanz) bzw. sehr flüssiger Konsistenz und hoher Abbaurate.

Bisherige Technologien geben Verweilzeiten des Gärsubstrats vor, welche die notwendige bakterielle Wachstumsrate sicher gestatten.

Ferner ist in der Druckschrift DE 10 2004 054 673 A1 eine Aufkonzentrierung der Biomasse vorgesehen, indem Suspension aus einer Zone des Biorektors mit geringer Feststoffkonzentration entnommen, eine Trennung von Gas und Feststoffen bewirkt und ein Teil der Feststoffe in den Bioreaktor zurückgeführt wird.

Mit der Patentanmeldung DE 198 13 022 A1 ist im Weiteren eine Immobilisierung von Mikroorganismen in Abwasser-Reinigungsanlagen durch Einsatz einer Matrix aus biologisch abbaubarem Biopolymer beschrieben.

Zum Stand der Technik gehört auch die Druckschrift DE 10 2006 035 794 A1. Gegenstand dieser Druckschrift ist ein Verfahren und eine Biogas-Anlage zur anaeroben Behandlung von zellstoffhaltigen Abfällen. Es wird offenbart, dass Stoffströme aus einer der Stufen mit steuerbarer Menge abziehbar und an einer anderen Stelle des Suspensionsströmungspfades zuführbar sind, wobei die abgezogenen Suspensionsanteile Schwimm- oder Sinkstoffe enthalten können. Eine sich auf oder unterhalb des oberen Substrat-Flüssigkeitsstandes und die gesamte Oberfläche umfassende Schwimmschicht, in der methanbildende Bakterien immobilisiert sind, wird dort nicht offenbart. Stufen meint in DE 10 2006 035 794 A1 die mechanische Aufbereitung, das Lösen in Prozesswasser, die Hygienisierungs-, biologische Aufbereitungs- bzw. Konfektionierstufe, folglich differenzierte Verfahrensstufen an unterschiedlichen Orten. Was In- und Output angeht, wird dort das Substrat dem liegenden Bioreaktor stirnseitig eingespeist und der Austritt von Materialien aus Gefäßen mittels Überläufen bewirkt bzw. werden die Gärprodukte in einem nachfolgenden separaten Behälter durch Sedimentation getrennt. Der Bioreaktor ist mit einer Vielzahl von Umwälz- und Kreislaufanschlüssen und einem Zu- sowie einem Ablauf versehen, die mit einer zentralen Dosierstation verbunden sind, um Durchmischung und Rückmischung zwischen Eintrags- und Austragsseite durchzuführen.

Ein Verfahren zur Biogaserzeugung und einen Gärbehälter dafür wird in der Offenlegungsschrift DE 10 2005 061 039 A1 beschrieben, wobei vergärbare Biomasse bewegt wird, sich eine Schwimmschicht auf der Biomasse bildet und entstehendes Biogas aufgefangen wird, wobei die Schwimmschicht in eine im wesentlichen stetige, laterale Bewegung in einer Richtung entlang des Gärbehälters gebracht wird, wobei am Ende des Gärbehälters die Schwimmschicht aufgelöst wird. Die Schwimmschicht wird gesteuert in eine definierte Bewegung versetzt. Dabei handelt es sich um eine "im wesentlichen stetige, laterale Bewegung entlang des Gärbehälters ..., wobei am Ende die Schwimmschicht aufgelöst wird" Dies wird dadurch bewirkt, dass auf dem oder nahe am Boden ein oder mehrere Strömungskanäle mitsamt Strömungserzeugungsmitteln vorgesehen sind, welche eine "gerichtete Rückströmung zur erforderlichen Strömungsbewegung unterhalb der Schwimmschicht in Richtung der lateralen Bewegung ermöglicht. Die Strömungsbahn dreht sich um horizontale und im wesentlichen senkrecht zur lateralen Bewegungsrichtung orientierte Achsen. Eine Drehbewegung der Schwimmschicht um eine vertikale Achse ist nicht offenbart worden.

In der deutschen Patentanmeldung DE 103 30 727 A1 wird ein Verfahren zur Erhöhung der Biogasausbeute aus organisch hochbelasteten Abwässern und flüssigen biogenen Suspensionen geringen Feststoffanteils durch anaerobe biologische Umsetzung erwähnt, wobei die am Umsetzungsprozess beteiligten Mikroorganismen durch regelmäßig wiederholten Substrat-Stress zu erhöhter Anpassung sowie erhöhter Stoffwechselaktivität gezwungen werden.

Der Erfindung lag die Aufgabe zugrunde, eine schnellere Zuführung neuen bzw. Abführung abgebauten Substrats in Biogasfermentern zu ermöglichen, ohne dass methanisierungsfähige Inhaltsstoffe und methanbildende Bakterien partiell mit ausgespült werden.

Die Aufgabe wurde gemäß den Merkmalen der Patentansprüche gelöst.

Die hier beschriebene Erfindung stellt eine - gegenüber dem Stand der Technik - andere neuartige Lösung dar. Dazu werden außer den für die Vergärung vorgesehenen Inputsubstraten grobfaserige schwimmschichtbildende Materialien - bereits durch Zumischen auf dem Pumpweg zum oder separat direkt in den Fermenter - zugegeben. Diese Materialien können sowohl natürlichen Ursprungs, wie etwa Stroh, oder industriell gefertigt sein, z.B. Gebilde aus Textil-, Kunststoff- bzw. ähnlichen Fäden oder Netze und Knäuel hieraus. Bevorzugt sind schwimmschichtfähige Stoffe organischen und durchaus im anaeroben Verfahren abbaubaren Ursprungs, da sie dadurch zum Teil positiven Einfluss auf das Fermentermilieu haben und außerdem durch Selbstabbau die Regelung der Schwimmschichtdimensionen, wie räumliche Ausdehnung und Dichte, erleichtern.

Auf Grund der Dichteunterschiede bildet sich auf dem Gärsubstrat eine aus dem grobfaserigen Material bestehende und die gesamte Oberfläche umfassende Schwimmschicht (1) aus. Deren Mächtigkeit wird durch gezielten Eintrag - bei Verwendung von Stroh auf eine Dicke von ca. drei bis etwa 40 Prozent der Fermenterfüllhöhe, bei alternativen Schichtmaterialien durchaus auch noch umfangreicher- gestaltet. Durch Entnahme lässt sich diese auch reduzieren. In dieser Schicht werden die für die Methanbildung relevanten Bakterienstämme dem Bedarf angepasst immobilisiert, reproduziert und deren Wirkungsbedingungen optimiert. Dazu wird neu zugeführtes Gärsubstrat von oben oder seitlich über eine Zuführungseinrichtung (2) in die Schwimmschicht eingebracht. Hierdurch bildet diese Schicht im Fermenter eine aktive Zone, in welcher Inputstoff hoher Konzentration, dagegen kaum bereits ausgegorenes Material, agglomeriert und energetisch umgesetzt wird. Je nach Menge und Geschwindigkeit zugeführter Substratmasse und abhängig von der Dicke der Faserschicht ist es möglich, dass sich letztgenannte zeitweilig oder ständig als ab- oder wieder aufschwebende bzw. in verschiedenen Höhen verharrende Schicht unterhalb des oberen Flüssigkeitsstandes darstellt. Das Verfahren ist sowohl auf mesophile als auch auf thermophile Temperaturbedingungen. (über 50 °C) ausgerichtet. Der aufrecht stehende Fermenter wird mit einer entsprechenden Heizung (3) versehen.

Um eine hochgradige biologische Prozesssteuerung zu erwirken, werden mittels spezieller Anordnungen von Vorrichtungen die jeweils aktuellen Verfahrenszustände überwacht und kontrolliert. Dazu werden zum einen Dicke und Lage der Schwimmschicht sowie die Beschaffenheit darunter- und ggf. darüberliegender Substratschichten über Sichtfenster, wie Bullaugen (4) bzw. Sehschlitze in der oberen Fermenterhälfte beobachtet oder bei beabsichtigter Fernüberwachung mittels anderer Messmethoden, z. B. Ultraschall, gemessen. Zum anderen sind für die Bestimmung des Temperaturverhaltens Sensoren vorgesehen, die unter Nutzung der eingearbeiteten, auf die Fermenterhöhe im gleichmäßigen Abstand verteilte Fühlerstutzen (5) differenziert zu messen ist. Schließlich sind, ebenfalls höhenverteilt, Stutzen für Probeentnahmen von Fermentersubstrat (6) vorhanden. Mittels Schnelltests vor Ort oder über Laboranalysen des in regelmäßigen Zeitabständen aufgenommenen Gärmaterials gestattet dies für unterschiedliche Substratschichtungen charakteristische Aussagen sowohl zum Säurestatus als auch zu den ggf. energetisch umsetzbaren oTS-Restgehalten (organische Trockensubstanz).

Die ermittelten Parameter bilden die Grundlage für zielgerichtete Reaktionen und Eingriffe in die Fermenterorganik. Die biologischen Prozesse werden erstens optimiert durch Einsatz eines Pumpsystems variabler Leistung, mit welchem - wahlweise permanent oder quasikontinuierlich - aus den analytisch ermittelten, brauchbaren Schichten unvollständig abgebautes Substrat am jeweils relevanten Abzugsstutzen (7) abgesaugt und durch Einsprühen über einen oder mehrere Spülanschlüsse (8) oberhalb der oder in die Faserschichtschicht erneut eingebracht wird. Diese lässt sich auf diese Weise energetisch zusätzlich anreichern und gleichzeitig in eine horizontale Drehbewegung versetzen. Die Drehung ist gewollt und notwendig, um das gesamte Volumen der Schwimmschicht bzw. oberhalb der Schwimmschicht mit dem neu eingebrachten Gärmaterial bzw. auch dem Rezirkulat zu versetzen.

Zweitens ermöglicht dieses Verfahren optimal zu bestimmen, in welchem Mischungsverhältnis die Komponenten Frischesubstrat und Fasermaterial bzw. reines Gärgut oder Schwimmschichtgut pur dem Fermenter neu zuzuführen sind.. Ist das Schwimmschichtmaterial natürlichen Ursprungs, wie z.B. Stroh, wird es über längere Zeit ebenfalls bakteriell umgesetzt und erhöht in diskretem Umfang die Gasausbeute. Die verbleibenden Fermentationsrückstände werden in der Regel zusammen mit dem abgebauten Substrat als Gärrest abgeführt. Sollte sich eine Reduzierung der Schwimmschichtdicke notwendig machen, ist dies auch über einen speziellen Abzug (9) realisierbar. Optional kann die Schwimmschicht im Falle einer unerwünschten Verdickung, Verfestigung oder Verfilzung auch mittels Rührwerk (10) aufgelöst werden. Ansonsten kann dieses mit gebotener Vorsicht außerdem genutzt werden, die horizontale Drehbewegung der Schwimmschichten mit zu unterstützen. Darüber hinaus ist jedoch ausdrücklich nicht erwünscht, eine Homogenisierung des Fermenterinhalts durch vertikales Durchmischen sämtlicher Inputmaterialien wie im konventionellen Biogasreaktor (Rührkesselfermenter) zu erwirken, und geht nur bedingt von statten. Vielmehr wird mit dem Verfahren und der Vorrichtung bewusst angestrebt, das Bestehen von Schichtungen auf Grund unterschiedlicher Masse- und Energiedichten des Fermenterinhalts auszunutzen, den Fermentationsprozess in einer Zone hoher biologischer Aktivität zu konzentrieren und hinsichtlich Raumbelastung, Substratabbau und Gasausbeute bestmögliche Resultate zu generieren.

Über den Eintrag einer einzelnen Schwimmschicht und die damit verbundenen Zonenbildung hinaus ist es durch Variationen von Schichtmaterialien differenzierter Dichte oder Nutzung unterschiedlicher Einbringungsorte bzw. -stellen möglich, im Fermenter mehrere Schichten auszubilden, sowohl unmittelbar aneinander grenzend als auch separat in verschiedenen Höhen. Dabei entstehen diverse Reaktionszonen für die Gärprozesse. Diese Zonen - was Milieubedingungen und biochemische Reaktionsabläufe betrifft - gezielt zu überwachen und durch planvolle Beschickung und Entnahme ausgegorenen Materialen bewusst zu beeinflussen, bewirkt eine weitgehende Optimierung der Gesamtprozesse im Hochleistungsreaktor.

Der Vorteil des erfindungsgemäßen Biogasfermenters gegenüber dem Stand der Technik ist die Nutzung von schwimmschichtfähigen Stoffen organischen und im anaeroben Verfahren abbaubaren Ursprungs zur Schwimmschichtausbildung, die in eine horizontale Drehbewegung versetzt werden, da sie dadurch positiven Einfluss auf das Fermentermilieu haben, außerdem durch Selbstabbau die Regelung der Schwimmschichtdimensionen erleichtern sowie die Gasausbeute erhöhen. Außerdem werden auf diese Weise physikalische Einrichtungen ersetzt.

Besonders vorteilhaft ist, dass eine Homogenisierung des Fermenterinhalts durch vertikales Durchmischen sämtlicher Inputmaterialien wie in konventionellen Biogasreaktoren nicht vorgesehen ist. Vielmehr wird mit dem Verfahren und der Vorrichtung bewusst angestrebt, das Bestehen von Schichtungen auf Grund unterschiedlicher Masse- und Energiedichten des Fermenterinhalts auszunutzen, den Fermentationsprozess in einer Zone hoher biologischer Aktivität zu konzentrieren und hinsichtlich Raumbelastung, Substratabbau und Gasausbeute bestmögliche Resultate zu generieren.

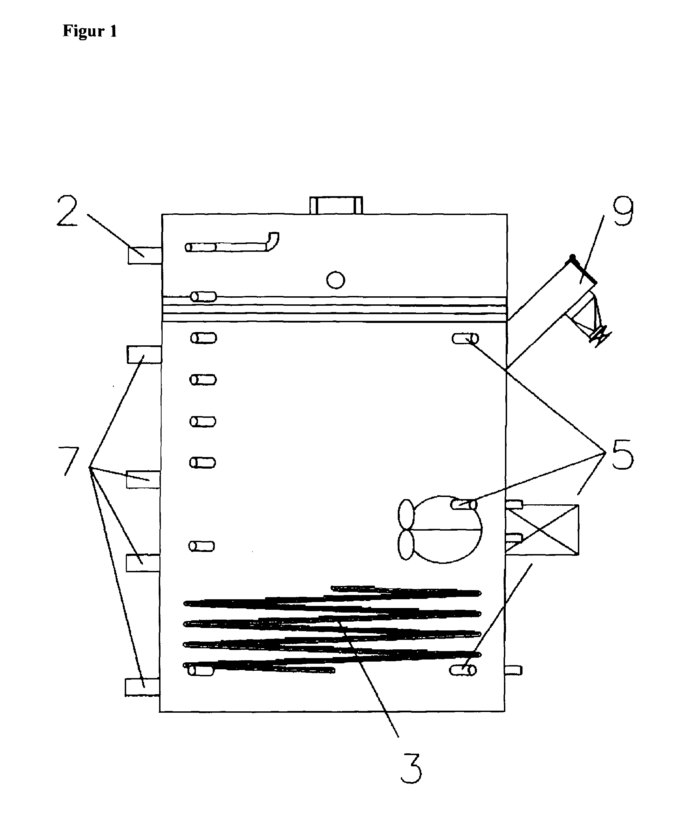

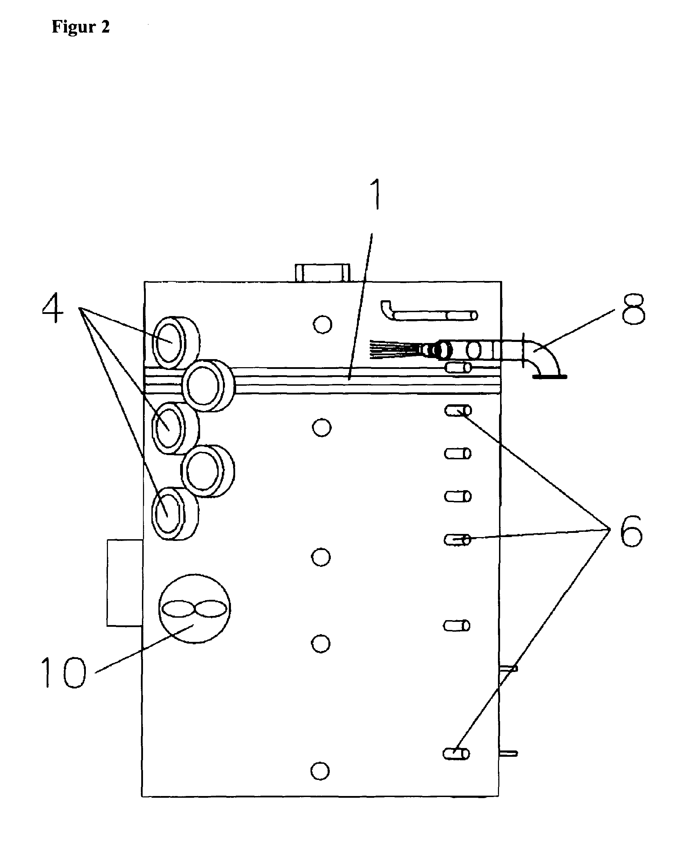

## Patentansprüche

1. Biogasfermenter, umfassend Einrichtungen und Substrate zur Erzeugung von Biogas, **dadurch gekennzeichnet, dass** sich auf oder unterhalb des oberen Substrat-Flüssigkeitsstandes eine die gesamte Oberfläche umfassende Schwimmschicht (1), die in eine horizontale Drehbewegung versetzt wird, befindet, in der methanbildende Bakterien immobilisiert sind.

2. Biogasfermenter nach Anspruch 1, **dadurch gekennzeichnet, dass** die Schwimmschicht (1) aus grobfaserigem Material besteht, das eine geringere Dichte als die Flüssigkeit mit dem Substrat aufweist.

3. Biogasfermenter nach Anspruch 1 oder 2, **dadurch gekennzeichnet, dass** die Schwimmschicht (1) aus Textil-, Kunststoff- bzw. ähnlichen Fäden, Netzen und Knäueln hieraus oder aus organischem Material besteht.

4. Biogasfermenter nach einem der Ansprüche 1 bis 3, **dadurch gekennzeichnet, dass** die Schwimmschicht (1) aus im anaeroben Verfahren abbaubarem Material, vorzugsweise Stroh, besteht.

5. Biogasfermenter nach einem der Ansprüche 1 bis 4, **dadurch gekennzeichnet, dass** er zusätzlich
a) eine Zuführungseinrichtung (2) zur Einbringung von Gärsubstrat in die Schwimmschicht (1) und / oder
b) Sichtfenster(4), vorzugsweise Bullaugen oder Sehschlitze, in der oberen Fermenterhälfte und / oder
c) Sensoren zur Fernüberwachung in der oberen Fermenterhälfte und / oder
d) Fühlerstutzen (5) und / oder Stutzen für Probeentnahmen von Fermentersubstrat und / oder
e) auf die Fermenterfülle verteilte Abzugsstutzen (7) zur Entnahme unvollständig abgebautem Substrats aus dafür geeigneten Schichten und / oder
f) Spülanschlüsse (8) oberhalb der oder in der Schwimmschicht (1) enthalten sind, um das aus brauchbaren Schichten entnommene, unvollständig abgebaute Material erneut in den Fermentationskreislauf einzubringen und / oder
g) Abzüge (9) und / oder Rührwerke (10) in ein- oder mehrfacher Ausfertigung enthält, um die Schwimmschicht hinsichtlich ihrer Dimension zu reduzieren oder um diese aufzulösen
enthält

6. Biogasfermenter nach Anspruch 5, **dadurch gekennzeichnet, dass** die Schwimmschicht (1) mittels der Spülanschlüsse (8) in eine horizontale Drehbewegung versetzt werden kann.

7. Verfahren zur Erhöhung der Besiedlungsdichte von methanbildenden Bakterienstämmen in Biogasfermentern mittels der Vorrichtung gemäß einem der Ansprüche 1 bis 6, **dadurch gekennzeichnet, dass** eine die gesamte Oberfläche umfassende Schwimmschicht (1), in der methanbildende Bakterien immobilisiert sind, auf oder unterhalb des oberen Substrat-Flüssigkeitsstandes eines Biogasfermenters gebracht wird.

8. Verfahren nach Anspruch 7, **dadurch gekennzeichnet, dass** die Dicke und Lage der Schwimmschicht (1) sowie die Beschaffenheit darunter- und / oder darüberliegender Substratschichten optisch oder instrumentell überwacht wird und daraus bestimmt wird, in welchem Mischungsverhältnis die Komponenten Frischesubstrat und Fasermaterial bzw. reines Gärgut oder Schwimmschichtgut dem Fermenter neu und / oder rezirkulierend zuzuführen sind.

9. Verfahren nach Anspruch 7 oder 8, **dadurch gekennzeichnet, dass** durch Variationen von Schichtmaterialien differenzierter Dichte oder Nutzung unterschiedlicher Einbringungsorte bzw. -stellen im Fermenter mehrere Schichten - sowohl unmittelbar aneinander grenzend als auch separat in verschiedenen Höhen - ausgebildet werden.

10. Verwendung des Biogasfermenters nach einem der Ansprüche 1 bis 6 zur Biogaserzeugung bei mesophilen als auch auf thermophilen (über 50 °C) Temperaturbedingungen.
